# FASCICULE DE BREVET EUROPEEN

(11) **EP 3 509 503 B1**
(45) Date de publication et mention de la délivrance du brevet: **27.12.2023**
(21) Numéro de dépôt: 17768184.8
(22) Date de dépôt: 01.09.2017
(51) Int. Cl.: A61B 17/04, A61B 17/062

(54) **PORTE-AIGUILLE ENDOSCOPIQUE**
ENDOSKOPISCHER NADELTRÄGER
ENDOSCOPIC NEEDLE CARRIER

(30) Priorité: 09.09.2016 FR 1658445
(43) Date de publication de la demande: 17.07.2019
(73) Titulaire: Institut Hospitalo-Universitaire de Chirurgie Mini -Invasive Guidee Par l'Image, 67091 Strasbourg (FR); IRCAD, Institut de Recherche Contre les Cancers de l'Appareil Digestif, 67091 Strasbourg (FR)
(72) Inventeur: HERNANDEZ, Juan, 67091 Strasbourg Cedex (FR); LEGNER, Andras, 67091 Strasbourg Cedex (FR)
(74) Mandataire: IP Trust
(86) Numéro de dépôt international: PCT/FR2017/052318
(87) Numéro de publication internationale: WO 2018/046822

(56) Documents cités:
- WO-A1-2011/055684
- DE-C1- 19 750 008
- US-A- 5 300 082
- US-A1- 2005 283 139
- US-A1- 2011 190 794
- US-A1- 2011 270 281

## Description

### Domaine de l'invention

La présente invention concerne le domaine des porte-aiguilles pour préhension et suture en chirurgie mini-invasive endoscopique ou coelioscopie.

De tels porte-aiguilles sont habituellement constitué par une pince présentant deux mors articulés et placée à l'extrémité d'un arbre. L'un des mors est dans une position fixe, l'autre est actionnée par la poignée de l'endoscope et présente une surface agrippante de l'aiguille.

En coelioscopie, la pince est utilisée seule ; en endoscopie flexible, elle est introduite au travers d'un endoscope flexible.

L'invention concerne plus particulièrement un porte-aiguille destiné à la manipulation endoscopique d'aiguilles de suture courbes, appliquée à l'endoscopie flexible, la laparoscopie et à des versions robotiques.

La manipulation de telles aiguilles présente des difficultés, même pour un chirurgien expérimenté, car les aiguilles courbes ont tendance à tourner à l'intérieur de la paire de mâchoires qui maintiennent l'aiguille.

### Etat de la technique

On connaît par exemple dans l'état de la technique le brevet US5257999 décrivant un porte-aiguille laparoscopique constitué par une tige et un ensemble de mâchoires commandé à une extrémité par un mécanisme d'ouverture et de fermeture. Les surfaces de préhension des mâchoires sont incurvées pour s'adapter à une aiguille chirurgicale courbée. Une mâchoire a une forme convexe tandis que l'autre mâchoire présente un rasage concave correspondant. La forme des surfaces de préhension des mâchoires incurvées oriente automatiquement l'aiguille chirurgicale courbée dans une position de suture souhaitée. La forme de l'ensemble de mâchoires permet également de libérer facilement et ressaisir l'aiguille chirurgicale courbée au cours du procédé de suture d'une opération laparoscopique.

On connaît aussi le brevet US8480689 décrivant un dispositif de suture pour la saisie d'une aiguille flexible pour adopter un état de repos incurvé.

On connaît aussi une solution décrite dans la demande de brevet américain US2011190794 concernant un porte-aiguille médicale constitué par une poignée disposée à l'extrémité proximale et un récepteur d'aiguille disposé à l'extrémité distale. Le récepteur de l'aiguille présente une première et une deuxième surface de serrage qui font face à des surfaces de serrage l'une vers l'autre et se prolongent dans une direction transversale à l'axe longitudinal du support. Les surfaces de serrage sont mobiles par rapport à l'autre par l'intermédiaire de la poignée, dans la direction de l'axe longitudinal du support.

La demande de brevet américain US2011270281 décrit un autre exemple de porte-aiguille présentant une partie de corps allongée ayant une extrémité proximale et une extrémité distale. Une tête rotative est configurée pour être disposée à l'extrémité distale de la partie formant corps et un élément d'actionnement configuré pour être disposé à l'extrémité proximale de la partie de corps. L'organe d'actionnement est en coopération fonctionnelle avec un ensemble de poignée pour permettre un mouvement de rotation de la tête rotative. La tête rotative coopère en outre avec une aiguille mobile par rapport à la tête rotative.

On connaît aussi dans l'état de la technique la demande de brevet internationale WO2011055684 décrivant un porte-aiguille pour la perforation ou la pénétration d'un tissu corporel au moyen d'une aiguille de suture, qui comprend une section poignée, la tige du porte-aiguille fournie devant la section poignée, une section glissière fournie devant la tige du porte-aiguille, et une tige de communication destinée à induire la communication entre la section poignée et la section glissière par l'intérieur de la tige du porte-aiguille, la section glissière ayant une surface de butée d'aiguille parallèle à et de la même forme que la surface d'extrémité de la tige du porte-aiguille sur le côté faisant face à la surface d'extrémité de la tige du porte-aiguille, un espace de fixation d'aiguille étant formé entre la surface de butée d'aiguille et la surface d'extrémité de la tige du porte-aiguille, et la tige de communication étant librement rétractée du côté de la section poignée, et extrudée après la rétraction par la manoeuvre de la section poignée, et la surface de butée d'aiguille se rapprochant de la surface d'extrémité de la tige du porte-aiguille par la rétraction de la tige de communication, l'espace de fixation d'aiguille s'en trouvant ainsi réduit, l'aiguille étant ainsi fixée entre la surface de butée d'aiguille et la surface d'extrémité de la tige du porte-aiguille, et la surface de butée d'aiguille étant séparée de la surface d'extrémité de la tige du porte-aiguille par l'extrusion de la tige de communication, et l'espace de fixation d'aiguille réduit est réinitialisé, l'aiguille fixée peut ainsi être détachée pour facilement et stablement fixer une aiguille de suture en chirurgie endoscopique, principalement à l'intérieur d'un corps de cavité à un angle prédéterminé.

On connaît aussi la demande de brevet US2005283130 qui concerne un appareil et un procédé de traitement de l'obésité. Un tube est positionné qui passe à travers la paroi abdominale du patient dans le système digestif supérieur du patient. Le patient est autorisé à mener ses activités quotidiennes, y compris à ingérer de la nourriture. Une fois que le patient a ingéré des aliments, ceux-ci sont extraits en les pompant par le tube digestif supérieur.

Le brevet américain US5300082 décrit un autre exemple connu d'instrument chirurgical ayant une poignée de commande pouvant être actionnée par un chirurgien pour saisir des aiguilles chirurgicales ou d'autres objets. L'instrument a une mâchoire mobile opposée à une mâchoire fixe. La poignée de commande met en prise un ressort de serrage qui ferme les mâchoires et exerce une force de préhension proportionnelle à la taille de l'objet situé entre les mâchoires.

Le brevet allemand DE19750008 décrit une autre solution encore d'un instrument chirurgical comporte un tube externe de section transversale interne rectangulaire, épousant la forme de l'insert en tant que guide à ajustement positif. Les extrémités du tube externe et de l'insert ont des composants fonctionnels qui se correspondent. L'instrument a une poignée latérale et un ressort de pression entre celle-ci et l'insert.

### Inconvénients de l'art antérieur

Avec les solutions de l'art antérieur, le chirurgien doit orienter angulairement les mors, généralement articulés par rapport à un pivot transversal, afin de faire rentrer l'aiguille entre les deux mors, puis refermer ces mors sur l'aiguille.

L'étape initiale d'orientation des mors, angulairement et en profondeur, peut être très délicate et nécessite parfois plusieurs dizaines de minutes de tâtonnement, notamment lorsque l'aiguille à saisir est dans une zone de faible visibilité endoscopique ou est positionnée de manière malcommode. Avec les solutions de l'art antérieur, la préhension de l'aiguille se fait avec une direction radiale précise, correspondant au plan de symétrie des mors et l'orientation dans ce plan de la ligne médiane à l'espace ouvert entre les deux mors. Même lorsque le chirurgien arrive à positionner angulairement et longitudinalement les mors par rapport à l'aiguille, il doit encore commander le déplacement radial pour positionner l'aiguille au bon niveau entre les mors. Ces opérations sont particulièrement délicates en chirurgie endoscopique, où l'aiguille n'est que difficilement visible et où le chirurgien n'a que peu d'information sur la progression de ses tâtonnements.

La solution proposée par la demande US201190794 ou US2011270281 n'est pas non plus satisfaisante car elle ne permet qu'un maintien très médiocre de l'aiguille, qui a tendance à se dégager dès que l'on exerce un effort pour la faire pénétrer dans un tissu.

### Solution apportée par l'invention

Afin de remédier à ces inconvénients, l'invention propose un porte-aiguille selon la revendication 1.

La première mâchoire est fixe en translation mais peut néanmoins présenter un degré de liberté en rotation autour de l'axe longitudinal, par rapport au tube. Elle est contrainte en translation par rapport au tube et contrainte en rotation par rapport à la seconde mâchoire (par l'intermédiaire d'une tige polygonale par exemple).

Selon la réalisation, les dents sont fixes par rapport à la mâchoire, ou escamotable élastiquement.

Selon une variante, ladite première mâchoire présente une surface frontale transversale munie d'une pluralité de dents périphériques s'étendant perpendiculairement à ladite surface frontale,
- ladite seconde mâchoire est constituée par une pièce cylindrique présentant une surface transversale munie d'une pluralité de dents périphériques
- lesdites mâchoires sont mobiles par un déplacement linéaire longitudinal entre une position au repos, où les surfaces frontales sont écartées pour délimiter entre les extrémités des dents un intervalle au moins égal à la section d'une aiguille et une position rapprochée où elles maintiennent l'aiguille courbée.

Avec la solution proposée par l'invention, l'extrémité de préhension présente une géométrie circulaire ne nécessitant plus une orientation radiale particulière par rapport à la position de l'aiguille. Quelque soit la position de l'aiguille dans le plan transversal passant entre les deux mâchoires, elle peut être accrochée par une ou plusieurs dents, et le rapprochement des mâchoires permet alors de la saisir et de la maintenir fermement.

Selon une variante, les dents de la seconde mâchoire sont décalées angulairement par rapport aux dents de la première mâchoire.

Selon l'invention, lesdites mâchoires présentent une alternance de dents périphériques et de rainures périphériques de formes et positions complémentaires aux dents de la mâchoire opposée.

Selon une variante avantageuse, les dites dents présentent dans le plan transversal une section transversale polygonale.

Selon un mode de réalisation particulier, chaque mâchoire présente trois dents et trois rainures pour recevoir les dents de la mâchoire complémentaire.

Selon une variante avantageuse, la surface extérieure des dents est semi-cylindrique.

Selon différentes variantes :
- la surface enveloppante desdites dents présente une section identique à la section desdites mâchoires. La surface extérieure prolonge ainsi le pourtour de la mâchoire correspondant pour présenter une surface périphérique dépourvue d'aspérités.
- la surface enveloppante desdites dents présente une section inférieure à la section desdites mâchoires. Dans ce cas, les dents sont en retrait du pourtour de la mâchoire correspondante, ce qui évite que les extrémités des dents ne viennent léser les tissus en contact avec l'extrémité de l'endoscope.
- la surface enveloppante desdites dents présente une section supérieure à la section desdites mâchoires. Dans ce cas, les dents débordent du pourtour de la mâchoire correspondante, ce qui favorise l'accrochage de l'aiguille courbée lorsqu'elle se trouve à proximité des mâchoires.

Selon une variante, ladite seconde mâchoire présente une extrémité hémisphérique. Elle facilite ainsi l'introduction du bout distal du porte-aiguille dans les organes creux.

Selon différentes variantes, la section desdites dents, dans un plan radial:
- présente une forme rectangulaire
- présente un bord intérieur incliné de façon à forcer le déplacement de l'aiguille pendant l'étape de fermeture des mâchoires
- présente un bord intérieur crénelé avec une largeur décroissante entre l'extrémité de la dent et la base de la dent
- présente une extrémité supérieure élargie.

Dans ces deux derniers cas, la variante améliore la retenue de l'aiguille engagée entre les deux mâchoires.

Selon une variante particulière, une partie au moins desdites dents sont déformables élastiquement, et présente une extrémité courbe.

Dans ce cas, ces dents sont rétractables et peuvent être effacées pour l'utilisation d'une aiguille de plus grande section ou en cas de positionnement particulier de l'aiguille.

Selon une variante, l'angle entre la dent et la surface de la mâchoire varie et/ou le profil de la dent varie.

Selon une variante de réalisation, lesdites dents sont formées par découpage de l'enveloppe tubulaire desdites mâchoires. Dans ce cas, la surface transversale est constituée par les arêtes des formes découpées.

Selon une variante particulière, ladite première mâchoire présente un canal central de section polygonal complémentaire de la section transversale de ladite âme mobile.

Selon une autre variante, certaines au moins desdites dents sont mobiles axialement.

Selon un mode de réalisation particulier, l'extrémité desdites dents présente un pan oblique dans le plan tangentiel de la mâchoire.

Avantageusement, chacune des mâchoires présentent des dents avec des pans obliques complémentaires et en ce ladite seconde mâchoire est libre en rotation par rapport à la première mâchoire et comporte un moyen élastique exerçant un couple dans le sens du rapprochement des pans obliques des dents complémentaires.

Selon une variante particulière, le porte-aiguille comporte une extrémité apte à être raccordée à un système motorisé.

### Description détaillée d'exemples non limitatifs de réalisation

La présente invention sera mieux comprise à la lecture de la description qui suit concernant plusieurs exemples non limitatifs de réalisation, se référant aux dessins annexés où :
- la figure 1 représente une vue de l'extrémité distale, de coté, d'un premier exemple de réalisation d'un porte-aiguille selon l'invention
- la figure 2 représente une vue de face des mâchoires portant une aiguille courbée selon ce premier exemple de réalisation d'un porte-aiguille selon l'invention
- la figure 3 représente une vue éclatée d'ensemble de ce premier exemple de réalisation d'un porte-aiguille selon l'invention
- la figure 4 représente une vue en perspective de trois-quarts arrière, de la seconde mâchoire de ce premier exemple de réalisation d'un porte-aiguille selon l'invention
- la figure 5 représente une vue en perspective de trois-quarts avant, de la première mâchoire de ce premier exemple de réalisation d'un porte-aiguille selon l'invention
- la figure 6 représente une vue en coupe transversale de la première mâchoire de ce premier exemple de réalisation d'un porte-aiguille selon l'invention
- la figure 7 représente une vue en perspective de trois-quarts avant, de la première mâchoire de ce premier exemple de réalisation d'un porte-aiguille selon l'invention
- la figure 8 représente une vue en perspective de trois-quarts arrière d'un porte-aiguille selon l'invention avec une aiguille courbe
- la figure 9 représente une vue de face d'un porte-aiguille selon l'invention avec une aiguille courbe
- les figures 10 et 11 représentent des vues en perspective d'un deuxième exemple de réalisation avec les mâchoires respectivement en position ouverte et en position fermées
- les figures 12 et 13 représentent des vues en perspective d'un troisième exemple de réalisation avec les mâchoires respectivement en position ouverte et en position fermées
- la figure 14 représente une vue en perspective d'un porte-aiguille selon un quatrième exemple de réalisation l'invention avec une aiguille courbe
- les figures 15 et 16 représentent des vues en perspective d'un cinquième exemple de réalisation avec les mâchoires respectivement en position ouverte et en position fermées
- les figures 17 à 20 représentent des vues de dessus de différentes alternatives de réalisation de l'implantation des dents
- les figures 21 à 26 représentent des vues selon un plan de coupe radial de différentes alternatives de réalisation des dents
- les figures 27 à 29 représentent des vues selon un plan de coupe transversal de différentes alternatives de réalisation des dents.

### Description détaillée d'un premier exemple de réalisation

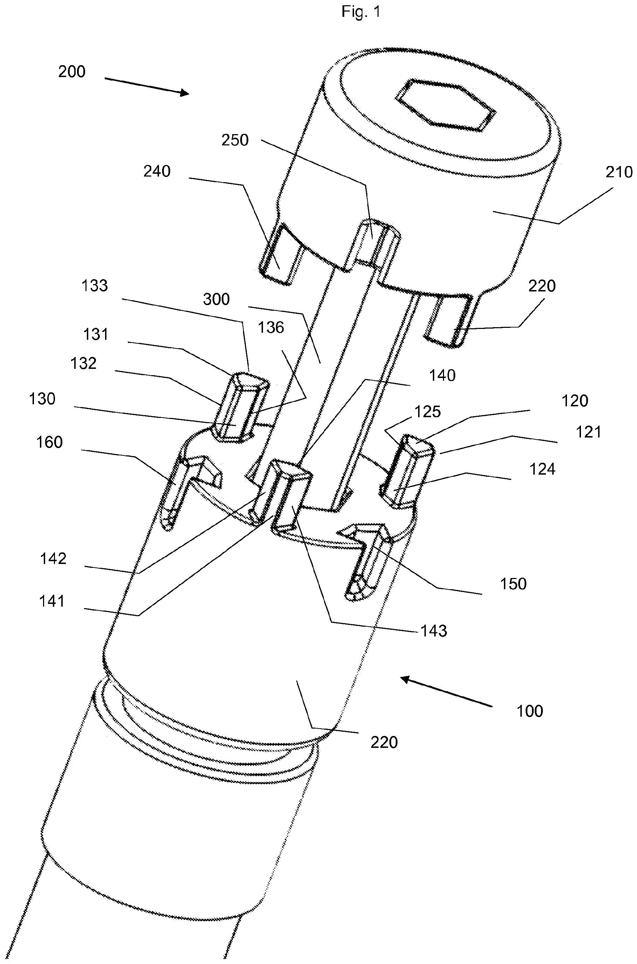

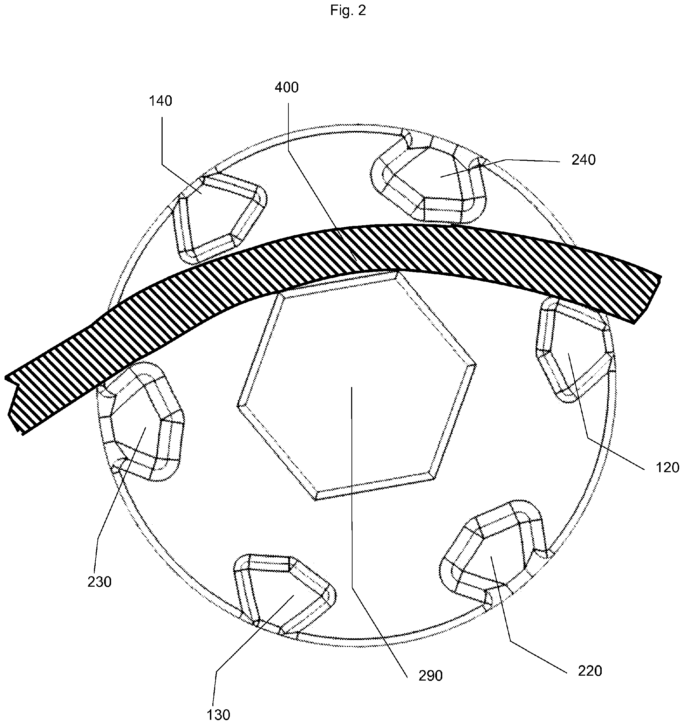

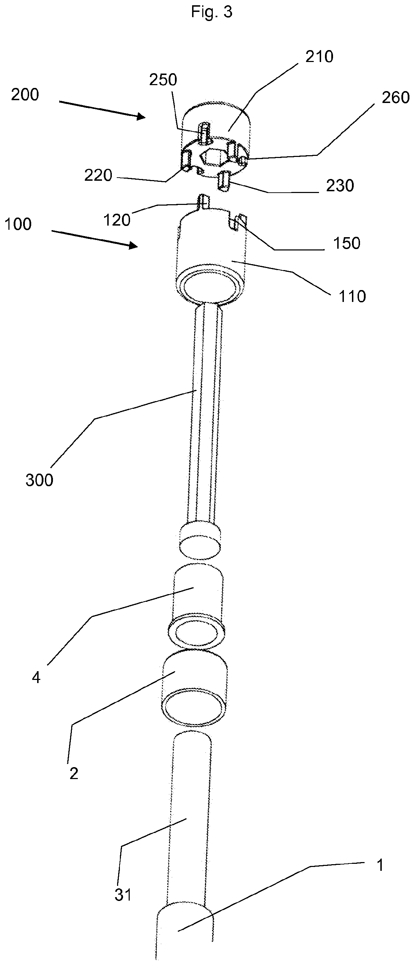

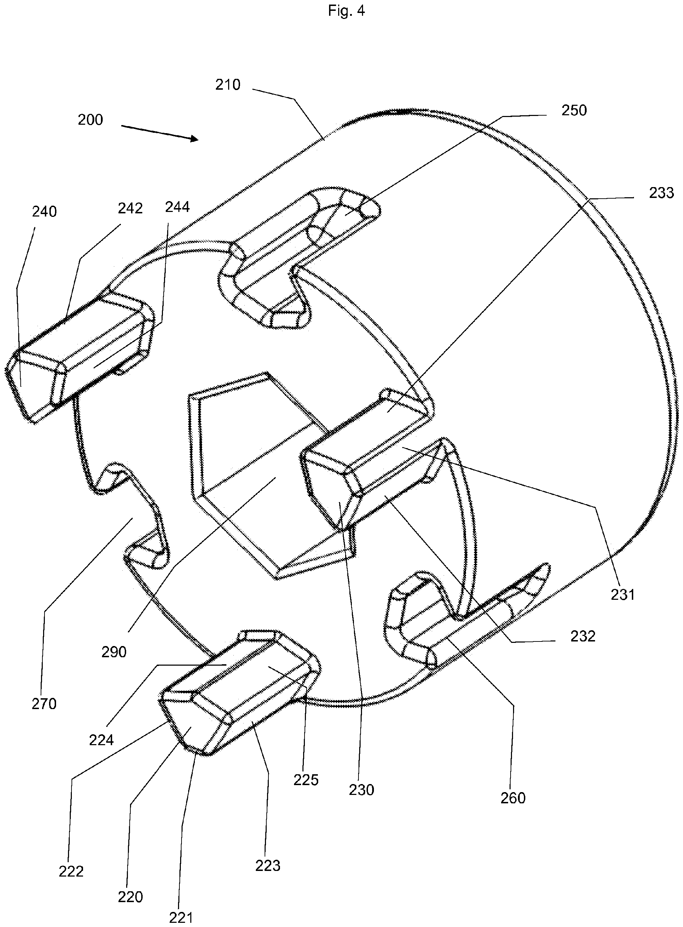

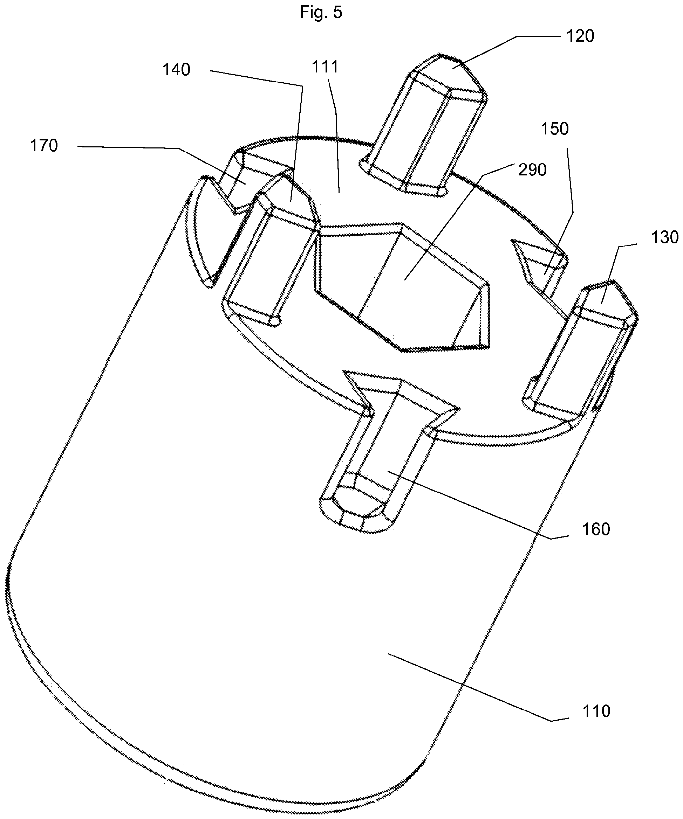

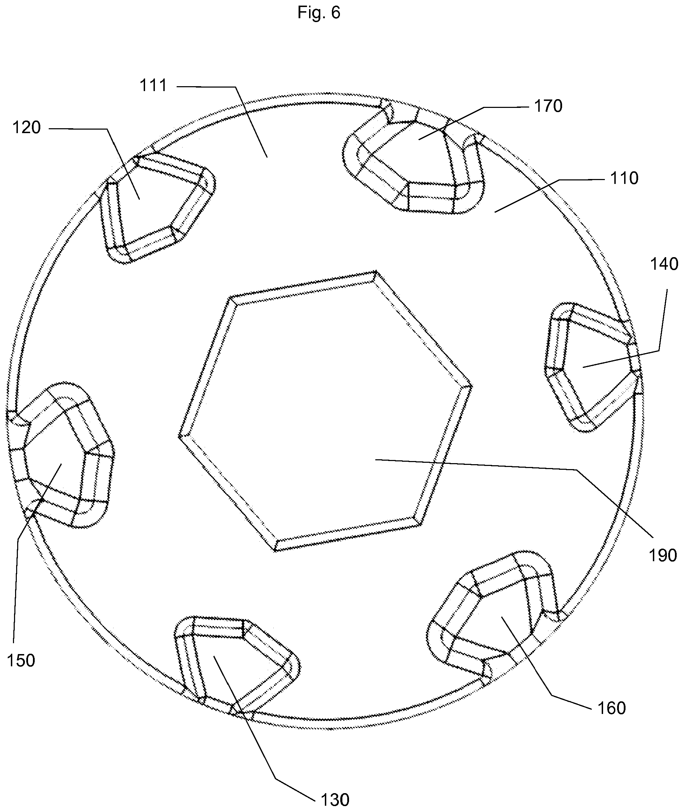

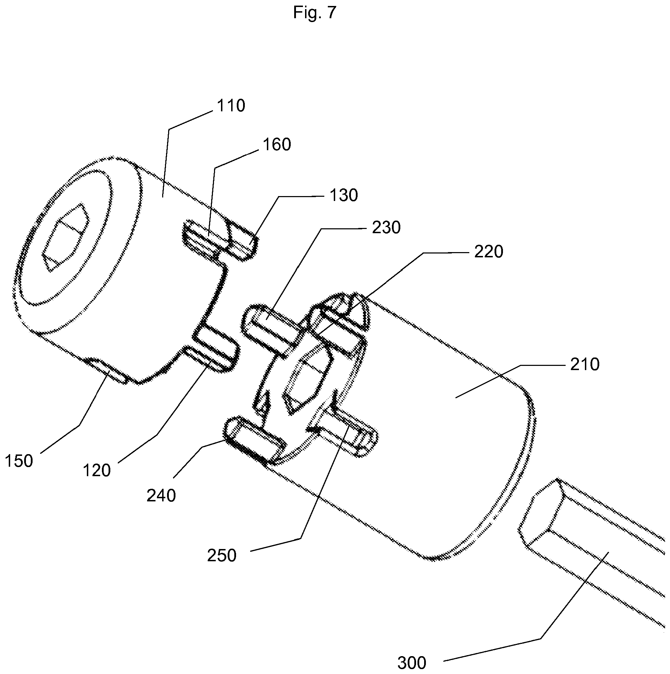

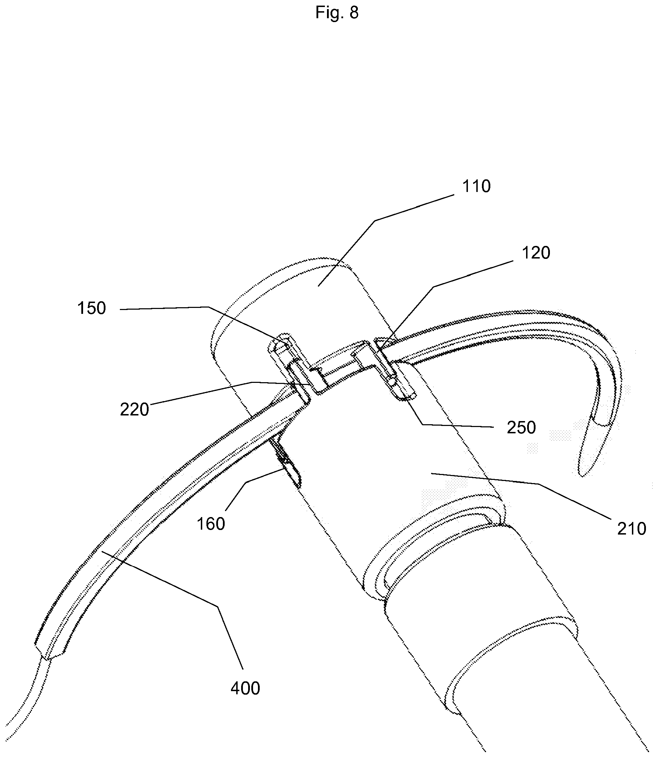

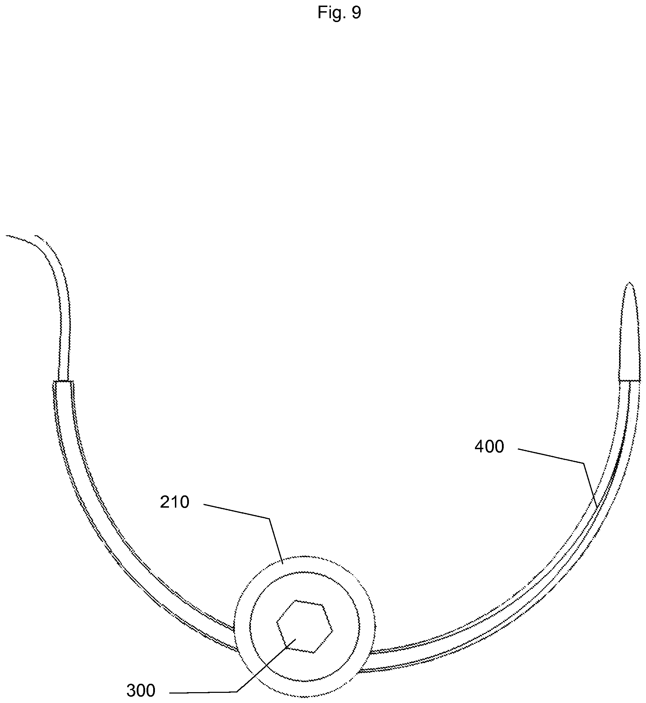

Le premier exemple de réalisation sera décrit de manière plus détaillée en référence aux figures 1 à 9.

Le porte-aiguille selon se premier exemple de réalisation est constitué d'une première mâchoire (100) et d'une seconde mâchoire (200) coaxiales avec une tige (300).

La première mâchoire (100) est montée sur une bague de connection (4) prévue à l'extrémité distale d'une tige (1) équipée elle-même d'une bague de connexion (2).

La première mâchoire (100) présente un corps cylindrique (110) métallique définissant une surface frontale (111) plane orientée en direction de la seconde mâchoire (200).

Ce corps cylindrique (110) est surmonté de trois dents (120, 130, 140) espacés angulairement de 120° et disposés à la périphérie de la surface frontale (111).

Ces dents (120, 130, 140) présentent une section transversale pentagonale, avec :
- des premiers côtés (121, 131, 141) semi-tubulaires pour prolonger la jupe périphérique du corps cylindrique (110),
- des deuxièmes et troisièmes côtés (142, 143) formant un angle compris entre 30 et 70° par rapport au plan tangentiel au corps cylindrique (110), de part et d'autre du plan radial
- des quatrièmes et cinquièmes côtés (124; 125) reliant les arêtes des deuxièmes et troisièmes côtés (122, 132, 143 ; 123, 133, 143) à une arête médiane respectivement (136).

Ces arêtes médianes (126, 136, 146) sont situées à l'intérieur de la seconde mâchoire, à l'opposé des premiers côtés (121, 131, 141). Le plan radial passe par ces arêtes médianes (136) et une ligne médiane des arêtes médianes (136).

La seconde mâchoire (200) présente une configuration similaire, avec trois dents (220, 230, 240) de même forme que les dents (120, 130, 140) de la première mâchoire (100), avec un décalage de 60° de façon à créer une alternance entre les dents fixes et les dents mobiles.

Elle présente une lumière centrale (290) de section polygonale complémentaire à la section transversale de la tige (300).

Le corps fixe (110) et le corps mobile (210) présente en regard de chaque dent de la mâchoire opposée des gorges (150, 160, 170 ; 250, 260, 270) dont la section transversale est complémentaire à la section transversale d'une dent de la mâchoire opposée et la profondeur adaptée pour permettre l'engagement d'une dent de la mâchoire opposée lorsque les mâchoires fixe (100) et mobile (200) sont en position rapprochée.

Le corps cylindrique (110) de la mâchoire (100) est traversé par un canal (190) de section hexagonale complémentaire à la section hexagonale de la tige (300) pour permettre le déplacement selon l'axe longitudinal de cette tige (300). Le corps (210) de la seconde mâchoire (200) est fixé sur l'extrémité de cette tige (300). L'orientation angulaire entre la première mâchoire (100) et la seconde mâchoire (200) est fixe dans ce premier exemple de réalisation.

Dans une réalisation où chacune des mâchoires présente trois dents, une aiguille courbe vient se loger entre les dents consécutives (230, 140, 240, 120) et la tige (300), et est calée contre les flans des dents et de la tige pour assurer un positionnement stable permettant ensuite de déplacer l'aiguille de manière parfaitement maîtrisée.

Les dents (220, 230, 240) présentent des sections transversales polygonales. Dans l'exemple décrit, elles présentent chacune cinq facettes (221 à 225, 231 à 232, 242, 244).

L'extrémité frontale du porte-aiguille peut comporter une poignée en forme de poire, permettant les gestes de :
- déplacement de la seconde mâchoire (200) en écartement ou rapprochement par rapport à la première mâchoire (100) par une pression sur la poignée.
- déplacement en rotation du porte-aiguille par une rotation de la poignée
- déplacement axial du porte-aiguille par un déplacement linéaire de la poignée.

Le déplacement du porte-aiguille est ainsi homothétique aux mouvements du chirurgien ce qui conduit à une manipulation très intuitive et précise.

Pour une variante coelioscopique la poignée aura une forme en « pistolet ». Pour une robotique, l'extrémité frontale du porte-aiguille ne comportera pas de poignée mais sera accouplée à un système motorisé.

### Description d'un deuxième exemple de réalisation

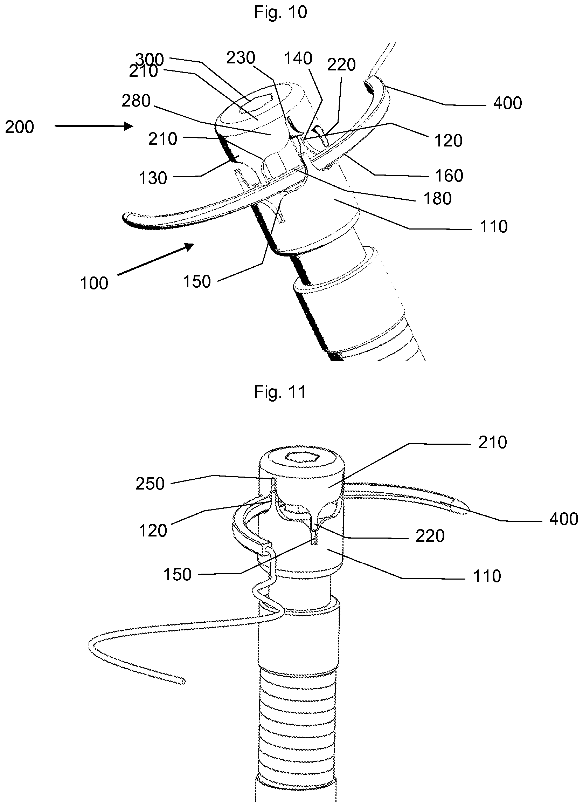

Les figures 10 et 11 illustrent un autre exemple de réalisation où les deux mâchoires fixe (100) et mobile (200) maintenant l'aiguille courbée (400) sont réalisées par découpage d'une tôle emboutie.

La première mâchoire (100) présente une jupe (110) tubulaire prolongée par des épanouissements (120, 130) formant les dents, et des découpes (150) de forme complémentaires des dents (220, 230) de la seconde mâchoire (200).

De la même façon, la seconde mâchoire (200) présente une jupe (210) tubulaire prolongée par des épanouissements (220, 230) formant les dents, et des découpes (250) de forme complémentaires des dents (120, 130) de la première mâchoire (100).

La zone de liaison entre une dent et une découpe présente un point d'inflexion avec une arête (180, 280) définissant la surface d'appui de l'aiguille (400), et remplissant la fonction de la surface transversale de la mâchoire de la première réalisation.

### Description d'un troisième exemple de réalisation

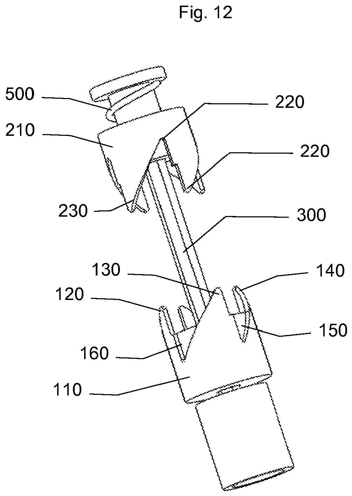

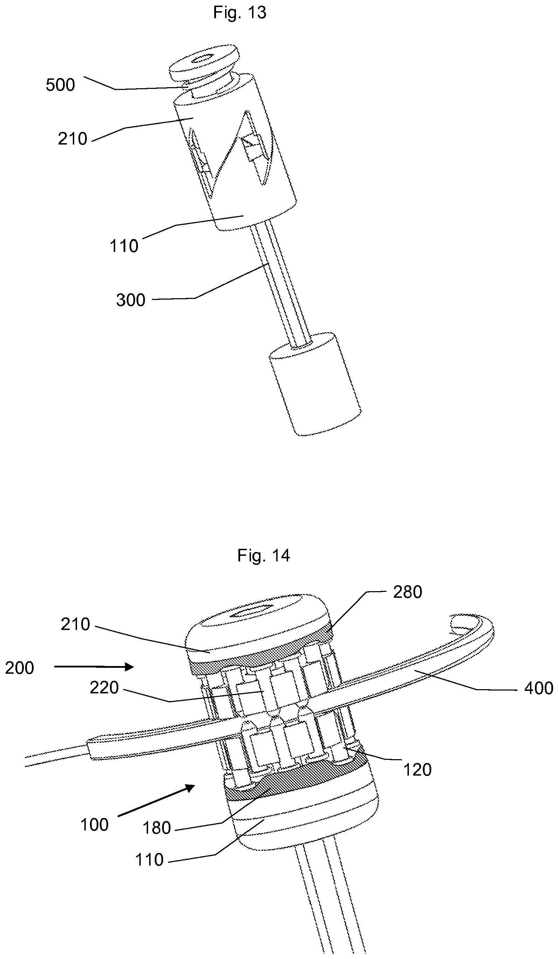

Les figures 12 et 13 illustrent un exemple de réalisation, les mâchoires présentent chacune quatre dents découpés dans un jupe tubulaire (110, 210), avec une section, dans un plan tangentiel, triangulaire, avec une alternance avec quatre découpes de forme complémentaire pour recevoir les dents de la mâchoire opposée.

La seconde mâchoire peut tourner angulairement autour de l'âme (300). Un ressort de rappel (500) tend à ramener les dents opposées les unes contre les autres.

Dans cette variante, les dents de la mâchoire supérieure ont une position initiale différente par rapport aux variantes précédemment décrites. Elles sont solidarisées à la seconde mâchoire (200) par le ressort (500). Lorsque la mâchoire se ferme, elle peut tourner en glissant contre les dents opposées jusqu'à maintenir l'aiguille entre les chants de la jupe. Quand on poursuit la pression, la partie cylindrique de la mâchoire continue à descendre jusqu'à serrer l'aiguille en haut et en bas. Cette configuration peut s'adapter à une pluralité d'aiguilles.

### Description d'un quatrième exemple de réalisation

La figure 14 représente une vue en perspective d'un quatrième exemple de réalisation avec les mâchoires en position fermées.

Les mâchoires (100, 200) présente des coussinets (180, 280) déformables élastiquement sur lesquels s'appuient les dents (220, 120).

Dans cette variante, les dents peuvent glisser dans les mâchoires (100, 200) pour s'adapter à une variété d'aiguilles. Lorsque les mâchoires se referment, certaines dents viennent en contact avec leur pendante sur la mâchoire opposée. Les dents qui entrent en contact avec l'aiguille se rétractent dans la mâchoire. Les coussinets (180, 280) sont constitués d'un matériau flexible (caoutchouc, silicone, ....) fournit une fonction de ressort à toutes les dents de façon à ce qu'elles soient toutes sorties quand les mâchoires sont ouvertes.

### Description d'un cinquième exemple de réalisation

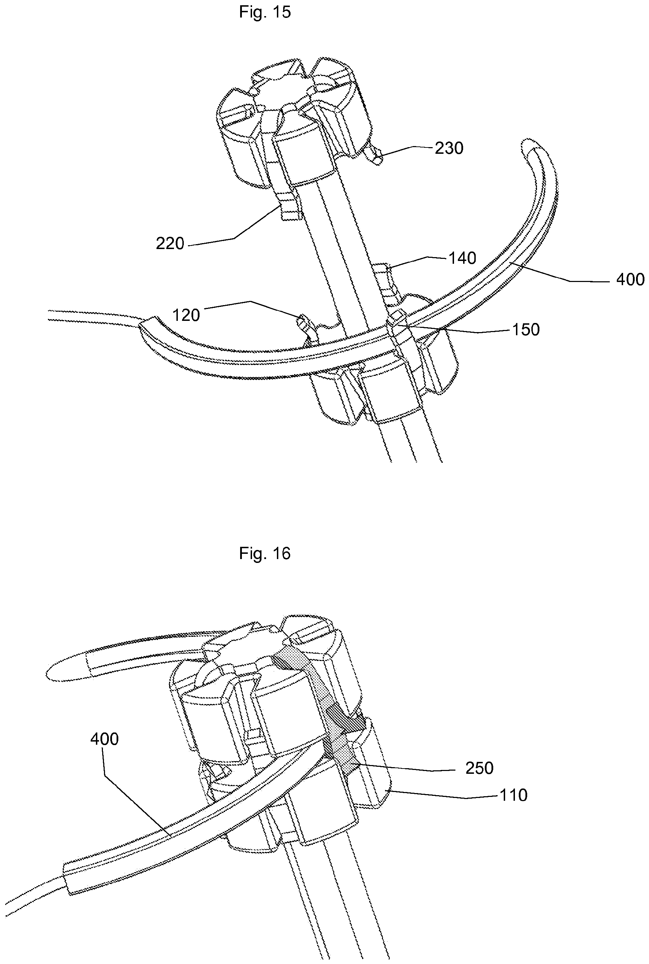

Les figures 15 et 16 représentent des vues en perspective d'un cinquième exemple de réalisation avec les mâchoires respectivement en position ouverte et en position fermées.

Selon cette variante, les dents (120, 130, 140, 220,230, 240) sont flexibles et viennent repousser l'aiguille (400) vers l'intérieur du fait de l'élasticité.

Dans cette variante, les dents peuvent subir une déformation élastique pour s'adapter à une variété d'aiguilles.

Lorsque les mâchoires se referment, certaines dents s'écartent et se positionnent sur la circonférence externe de l'aiguille, tout en restant canalisées dans la rainure de la mâchoire opposée.

### Implantation des dents

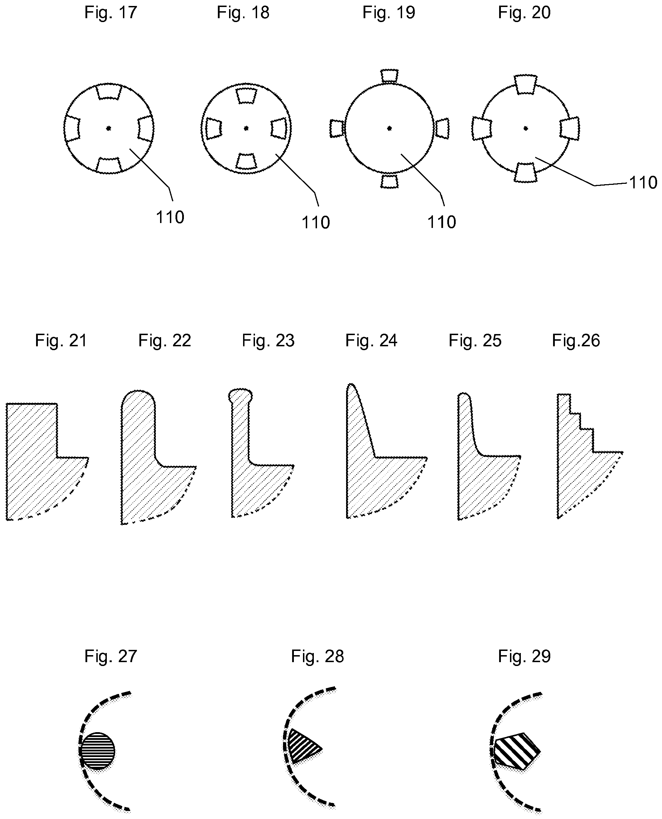

Les figures 17 à 20 représentent des vues de dessus de différentes alternatives de réalisation de l'implantation des dents.

Les dents peuvent être disposées à la bordure de la mâchoire comme illustré par la figure 17. Dans ce cas, le bord extérieure des dents s'inscrit dans le prolongement du corps de la mâchoire sans aspérité ni creux.

Elles peuvent aussi être disposées en retrait de la bordure de la mâchoire comme représenté en figure 18. Dans ce cas elles réduisent l'encombrement latéral.

Elles peuvent aussi être disposées à l'extérieur de la bordure de la mâchoire, comme représenté en figure 19, afin d'offrir plus d'accrochage d'une aiguille placée à proximité des mâchoires.

Elles peuvent encore être disposées à cheval sur la bordure de la mâchoire comme illustré par la figure 20, pour un compromis entre un accrochage amélioré de l'aiguille et un encombrement latéral restreint.

### Section des dents

Les figures 21 à 26 représentent des vues selon un plan de coupe radial de différentes alternatives de réalisation des dents.

Les dents peuvent présenter dans le plan transversal radial une section :
- rectangulaire, avec un sommet plat ou chanfreiné, comme représenté en figure 21
- Rectangulaire avec un sommet arrondi comme représenté en figure 22
- Rectangulaire, avec un sommet évasé comme représenté en figure 23 pour améliorer la retenue de l'aiguille
- triangulaire, comme représenté en figure 24 pour ramener l'aiguille contre l'âme centrale lors de la fermeture des mâchoires
- trapézoïdale avec une largeur croissante vers la base de la dent, avec un bord intérieur incurvé comme représenté en figure 25, pour assurer un guidage de l'aiguille vers le centre pendant la fermeture des mâchoires
- crénelée comme représenté en figure 26 pour permettre une adaptation à différentes tailles d'aiguilles.

### Section transversale des dents

Les figures 27 à 29 représentent des vues selon un plan de coupe transversal de différentes alternatives de réalisation des dents.

La section peut être :
- circulaire comme représentée en figure 27
- triangulaire avec une base arrondie comme représentée en figure 28, pour présenter une arête centrale d'appui contre la surface de l'aiguille
- pentagonale comme représentée en figure 29.

## Revendications

1. - Porte-aiguille endoscopique pour la manipulation d'aiguilles courbées comprenant une âme mobile (300) en translation, un mécanisme d'actionnement à distance prévu à l'une des extrémités du porte aiguille, et à l'autre extrémité un ensemble articulé comprenant une première mâchoire de préhension (100) solidaire d'un conduit et une seconde mâchoire de préhension (200) actionnée par ladite âme mobile (300):
- lesdites mâchoires (100, 200) sont constituées par des pièces cylindriques coaxiales avec ladite âme mobile (300)
- ladite première mâchoire (100) présente une pluralité de dents périphériques (120, 130, 140) s'étendant longitudinalement,
- ladite seconde mâchoire (200) est constituée par une pièce cylindrique présentant une pluralité de dents périphériques (220, 230, 240)
- la dite seconde mâchoire (200) est mobile par rapport à la première mâchoire (100) par un déplacement linéaire longitudinal entre une position au repos, où les mâchoires (100, 200) sont écartées pour délimiter entre les extrémités des dents un intervalle au moins égal à la section d'une aiguille (400) et une position rapprochée où elles maintiennent l'aiguille courbée (400),
**caractérisé en ce que**
lesdites mâchoires (100, 200) présentent une alternance desdites dents périphériques et de rainures périphérique: (150, 160, 170 ; 250, 260, 270) de formes et positions complémentaires aux dents de la mâchoire opposée.

2. - Porte-aiguille endoscopique selon la revendication 1 **caractérisé en ce que** les dents périphériques (220, 230, 240) de ladite seconde mâchoire (200) sont décalées angulairement par rapport aux dents (120, 130, 140) de ladite première mâchoire (100)

3. - Porte-aiguille endoscopique selon la revendication 1 **caractérisé en ce que** les dites dents présentent dans le plan transversal une section transversale polygonale.

4. - Porte-aiguille endoscopique selon la revendication 1 **caractérisé en ce que** chaque mâchoire présente trois dents et trois rainures pour recevoir les dents de la mâchoire complémentaire.

5. - Porte-aiguille endoscopique selon la revendication 1 **caractérisé en ce que** la surface extérieure des dents est semi-cylindrique.

6. - Porte-aiguille endoscopique selon la revendication 1 **caractérisé en ce que** la surface enveloppante desdites dents présente une section identique à la section desdites mâchoires.

7. - Porte-aiguille endoscopique selon la revendication 1 **caractérisé en ce que** la surface enveloppante desdites dents présente une section inférieure à la section desdites mâchoires.

8. - Porte-aiguille endoscopique selon la revendication 1 **caractérisé en ce que** la surface enveloppante desdites dents présente une section supérieure à la section desdites mâchoires.

9. - Porte-aiguille endoscopique selon la revendication 1 **caractérisé en ce que** ladite seconde mâchoire présente une extrémité hémisphérique.

10. - Porte-aiguille endoscopique selon la revendication 1 **caractérisé en ce que** la section desdites dents, dans un plan radial, est rectangulaire.

11. - Porte-aiguille endoscopique selon la revendication 1 **caractérisé en ce que** la section desdites dents, dans un plan radial, présente un bord intérieur incliné.

12. - Porte-aiguille endoscopique selon la revendication 1 **caractérisé en ce que** la section desdites dents, dans un plan radial, présente un bord intérieur crénelé avec une largeur décroissante entre l'extrémité de la dent et la base de la dent.

13. - Porte-aiguille endoscopique selon la revendication 1 **caractérisé en ce que** la section desdites dents, dans un plan radial, présente une extrémité supérieure élargie.

14. - Porte-aiguille endoscopique selon la revendication 1 **caractérisé en ce qu'**une partie au moins desdites dents sont déformables élastiquement, et présente une extrémité évasée.

15. - Porte-aiguille endoscopique selon la revendication 1 **caractérisé en ce que** lesdites dents sont formées par découpage de l'enveloppe tubulaire desdites mâchoires.

16. - Porte-aiguille endoscopique selon la revendication 1 **caractérisé en ce que** ladite première mâchoire présente un canal central de section polygonal complémentaire de la section transversale de ladite âme mobile.

17. - Porte-aiguille endoscopique selon la revendication 1 **caractérisé en ce que** certaines au moins desdites dents sont mobiles axialement.

18. - Porte-aiguille endoscopique selon la revendication 1 **caractérisé en ce que** l'extrémité desdites dents présente un pan oblique dans le plan tangentiel de la mâchoire.

19. - Porte-aiguille endoscopique selon la revendication précédente **caractérisé en ce que** chacune des mâchoires présente des dents avec des pans obliques complémentaires et en ce ladite seconde mâchoire est libre en rotation par rapport à la première mâchoire et comporte un moyen élastique exerçant un couple dans le sens du rapprochement des pans obliques des dents complémentaires.

20. - Porte-aiguille endoscopique selon la revendication 1 **caractérisé en ce qu'**il comporte une extrémité apte à être raccordée à un système motorisé.

## Patentansprüche

1. Endoskopischer Nadelträger für den Umgang mit gekrümmten Nadeln, umfassend einen translatorisch bewegbaren Kern (300), einen Fernbetätigungsmechanismus, der an einem der Enden des Nadelträgers vorgesehen ist, und an dem anderen Ende eine Gelenkanordnung, umfassend eine erste Greifbacke (100), die mit einer Leitung fest verbunden ist, und eine zweite Greifbacke (200), die durch den bewegbaren Kern (300) betätigt wird:
- wobei die Backen (100, 200) aus zylindrischen Stücken, die mit dem bewegbaren Kern (300) koaxial sind, bestehen
- wobei die erste Backe (100) eine Vielzahl von Umfangszähnen (120, 130, 140), die sich in Längsrichtung erstrecken, vorweist,
- wobei die zweite Backe (200) aus einem zylindrischen Stück, das eine Vielzahl von Umfangszähnen (220, 230, 240) vorweist, besteht
- wobei die zweite Backe (200) relativ zu der ersten Backe (100) durch eine lineare Längsverschiebung zwischen einer Ruheposition, in der die Backen (100, 200) zum Begrenzen zwischen den Enden der Zähne eines Intervalls, das mindestens gleich dem Abschnitt einer Nadel (400) ist, auseinanderstehend sind, und einer angenäherten Position, in der sie die gekrümmte Nadel (400) halten, bewegbar ist,
**dadurch gekennzeichnet, dass:**
die Backen (100, 200) einen Wechsel der Umfangszähne und von Umfangsnuten (150, 160, 170; 250, 260, 270) mit Formen und Positionen, die komplementär zu den Zähnen der gegenüberliegenden Backe sind, vorweisen.

2. Endoskopischer Nadelträger nach Anspruch 1,
**dadurch gekennzeichnet, dass** die Umfangszähne (220, 230, 240) der zweiten Backe (200) relativ zu den Zähnen (120, 130, 140) der ersten Backe (100) winkelversetzt sind.

3. Endoskopischer Nadelträger nach Anspruch 1,
**dadurch gekennzeichnet, dass** die Zähne in der Querebene einen polygonalen Querabschnitt vorweisen.

4. Endoskopischer Nadelträger nach Anspruch 1,
**dadurch gekennzeichnet, dass** jede Backe drei Zähne und drei Nuten zum Aufnehmen der Zähne der komplementären Backe vorweist.

5. Endoskopischer Nadelträger nach Anspruch 1,
**dadurch gekennzeichnet, dass** die Außenoberfläche der Zähne halbzylindrisch ist.

6. Endoskopischer Nadelträger nach Anspruch 1,
**dadurch gekennzeichnet, dass** die umhüllende Oberfläche der Zähne einen Abschnitt, der identisch mit dem Abschnitt der Backen ist, vorweist.

7. Endoskopischer Nadelträger nach Anspruch 1,
**dadurch gekennzeichnet, dass** die umhüllende Oberfläche der Zähne einen Abschnitt, der kleiner als der Abschnitt der Backen ist, vorweist.

8. Endoskopischer Nadelträger nach Anspruch 1,
**dadurch gekennzeichnet, dass** die umhüllende Oberfläche der Zähne einen Abschnitt, der größer als der Abschnitt der Backen ist, vorweist.

9. Endoskopischer Nadelträger nach Anspruch 1,
**dadurch gekennzeichnet, dass** die zweite Backe ein halbkugelförmiges Ende vorweist.

10. Endoskopischer Nadelträger nach Anspruch 1,
**dadurch gekennzeichnet, dass** der Abschnitt der Zähne, in einer radialen Ebene, rechteckig ist.

11. Endoskopischer Nadelträger nach Anspruch 1,
**dadurch gekennzeichnet, dass** der Abschnitt der Zähne, in einer radialen Ebene, einen geneigten Innenrand vorweist.

12. Endoskopischer Nadelträger nach Anspruch 1,
**dadurch gekennzeichnet, dass** der Abschnitt der Zähne, in einer radialen Ebene, einen zackigen Innenrand mit einer abnehmenden Breite zwischen dem Ende des Zahns und der Basis des Zahns vorweist.

13. Endoskopischer Nadelträger nach Anspruch 1,
**dadurch gekennzeichnet, dass** der Abschnitt der Zähne, in einer radialen Ebene, ein erweitertes oberes Ende vorweist.

14. Endoskopischer Nadelträger nach Anspruch 1,
**dadurch gekennzeichnet, dass** ein Teil mindestens der Zähne elastisch verformbar sind und ein ausgeweitetes Ende vorweist.

15. Endoskopischer Nadelträger nach Anspruch 1,
**dadurch gekennzeichnet, dass** die Zähne durch Zerschneiden der röhrenförmigen Hülle der Backen ausgebildet werden.

16. Endoskopischer Nadelträger nach Anspruch 1,
**dadurch gekennzeichnet, dass** die erste Backe einen zentralen Kanal mit polygonalem Abschnitt, der komplementär zu dem Querabschnitt des bewegbaren Kerns ist, vorweist.

17. Endoskopischer Nadelträger nach Anspruch 1,
**dadurch gekennzeichnet, dass** mindestens gewisse Zähne axial bewegbar sind.

18. Endoskopischer Nadelträger nach Anspruch 1,
**dadurch gekennzeichnet, dass** das Ende der Zähne eine Schräge in der Tangentialebene der Backe vorweist.

19. Endoskopischer Nadelträger nach dem vorstehenden Anspruch,
**dadurch gekennzeichnet, dass** jede der Backen Zähne mit komplementären Schrägen vorweist, und dass die zweite Backe relativ zu der ersten Backe frei drehbar ist und ein elastisches Mittel aufweist, das ein Drehmoment in der Annäherungsrichtung der Schrägen der komplementären Zähne ausübt.

20. Endoskopischer Nadelträger nach Anspruch 1,
**dadurch gekennzeichnet, dass** er ein Ende aufweist, das geeignet ist, um mit einem motorisierten System verbunden zu werden.

## Claims

1. Endoscopic needle holder for handling curved needles comprising a translatable core (300), a remote actuation mechanism provided at one of the ends of the needle door, and at the other end an articulated assembly comprising a first gripping jaw (100) secured to a duct and a second gripping jaw (200) actuated by said movable core (300):
- said jaws (100, 200) consist of cylindrical parts which are coaxial with said movable core (300)
- said first jaw (100) has a plurality of peripheral teeth (120, 130, 140) which extend longitudinally,
- said second jaw (200) consists of a cylindrical part having a plurality of peripheral teeth (220, 230, 240)
- said second jaw (200) is movable relative to the first jaw (100) by a longitudinal linear movement between a rest position in which the jaws (100, 200) are spaced apart to delimit, between the ends of the teeth, an interval which is at least equal to the cross-section of a needle (400), and a close position in which they support the curved needle (400),
**characterized in that**
said jaws (100, 200) have alternating peripheral teeth and peripheral grooves (150, 160, 170; 250, 260, 270) having shapes and positions which are complementary to the teeth of the opposite jaw.

2. Endoscopic needle holder according to claim 1, **characterize in that** the peripheral teeth (220, 230, 240) of said second jaw (200) are angularly offset relative to the teeth (120, 130, 140) of said first jaw (100)

3. Endoscopic needle holder according to claim 1, **characterized in that** said teeth have a polygonal cross-section in the transverse plane.

4. Endoscopic needle holder according to claim 1, **characterized in that** each jaw has three teeth and three grooves to receive the teeth of the complementary jaw.

5. Endoscopic needle holder according to claim 1, **characterized in that** the exterior surface of the teeth is semi-cylindrical.

6. Endoscopic needle holder according to claim 1, **characterized in that** the enveloping surface of said teeth has a cross-section which is identical to the cross-section of said jaws.

7. Endoscopic needle holder according to claim 1, **characterize in that** the enveloping surface of said teeth has a cross-section which is smaller than the cross-section of said jaws.

8. Endoscopic needle holder according to claim 1, **characterize in that** the enveloping surface of said teeth has a cross-section which is greater than the cross-section of said jaws.

9. Endoscopic needle holder according to claim 1, **characterized in that** said second jaw has a hemispherical end.

10. Endoscopic needle holder according to claim 1, **characterize in that** the cross-section of said teeth, in a radial plane, is rectangular.

11. Endoscopic needle holder according to claim 1, **characterize in that** the cross-section of said teeth, in a radial plane, has an inclined inner edge.

12. Endoscopic needle holder according to claim 1, **characterize in that** the cross-section of said teeth, in a radial plane, has a crenelated inner edge with a width which decreases between the end of the tooth and the base of the tooth.

13. Endoscopic needle holder according to claim 1, **characterize in that** the cross-section of said teeth, in a radial plane, has a widened upper end.

14. Endoscopic needle holder according to claim 1, **characterize in that** at least part of said teeth is elastically deformable, and has a flared end.

15. Endoscopic needle holder according to claim 1, **characterized in that** said teeth are formed by cutting the tubular casing of said jaws.

16. Endoscopic needle holder according to claim 1, **characterized in that** said first jaw has a central channel with a polygonal cross-section which is complementary to the cross-section of said mobile core.

17. Endoscopic needle holder according to claim 1, **characterized in that** at least some of said teeth are axially movable.

18. Endoscopic needle holder according to
claim 1, **characterized in that** the end of said teeth has an oblique face in the tangential plane of the jaw.

19. Endoscopic needle holder according to the preceding claim,
**characterized in that** each of the jaws has teeth with complementary oblique faces and **in that** said second jaw is free to rotate relative to the first jaw and comprises a resilient means which exerts a torque in the direction of convergence of the oblique faces of the complementary teeth.

20. Endoscopic needle holder according to claim 1, **characterized in that** it comprises an end which is capable of being connected to a motor-driven system.
